## Europäisches Patentamt

## European Patent Office

## Office européen des brevets

(19)

(11) Numéro de publication: **0 151 326**
**B1**

(12) **FASCICULE DE BREVET EUROPÉEN**

(45) Date de publication du fascicule du brevet:
12.07.89

(21) Numéro de dépôt: 84201920.0

(22) Date de dépôt: 19.12.84

(51) Int. Cl.⁴: **A 61 K 31/57**, A 61 K 31/135 //
(A61K31/57, 31:135)

(54) **Médicament antioestrogène pour administration percutanée.**

(30) Priorité: 20.01.84 FR 8400927

(43) Date de publication de la demande:
14.08.85 Bulletin 85/33

(45) Mention de la délivrance du brevet:
12.07.89 Bulletin 89/28

(84) Etats contractants désignés:
IT

(56) Documents cité:
FR-A-2 515 041

CHEMICAL ABSTRACTS, vol. 96, no. 9, 1 mars 1982,
page 29, no. 62664k, Columbus, Ohio, US; M.
SLUYSER et al.: "Effect of monohydroxytamoxifen
on mouse mammary tumors" & EUR. J. CANCER
CLIN. ONCOL. 1981, 17(9), 1063-5
CHEMICAL ABSTRACTS, vol. 85, no. 3, 19 juillet
1976, page 81, no. 14290m, Columbus, Ohio, US; S.
SEKIYA et al.: "The combined effect of
nonsteroidal anti-estrogens and sex steroids on the
growth of rat uterine adenocarcinoma cells in
tissue culture" & BR. J. OBSTET. GYNAECOL. 1976,
83(3), 183-6
J. of Clinical Endocrinology and Metabolism 63(5)
p.1174(1986)
J. of Clinical Endocrinology and Metabolism 114(5)
p.1483(1984)

(73) Titulaire: **Mauvais-Jarvis, Pierre, 12 Parc de Béarn,
F-92210 Saint-Cloud (Hauts de Seine) (FR)**
Titulaire: **Kuttenn, Frédérique, 6, Avenue des
Gobelins, F-75005 Paris (Seine) (FR)**

(72) Inventeur: **Mauvais-Jarvis, Pierre, 12 Parc de
Béarn, F-92210 Saint-Cloud (Hauts de Seine) (FR)**
Inventeur: **Kuttenn, Frédérique, 6, Avenue des
Gobelins, F-75005 Paris (Seine) (FR)**

(74) Mandataire: **Van Malderen, Michel, p.a. Freylinger
& Associés 22 avenue J.S. Bach (bte 43), B-1080
Bruxelles (BE)**

(56) Documents cité: (suite)

Le dossier contient des informations techniques
présentées postérieurement au dépôt de la
demande et ne figurant pas dans le présent
fascicule.

## Description

La présente invention concerne un médicament antioestrogène applicable notamment au traitement de certaines formes de tumeurs, en particulier de la glande mammaire dans ses formes hormonodépendantes.

On connaît à l'heure actuelle un antioestrogène administrable par voie orale, dit tamoxifène, et constitué par le 1(p-ß-diméthylaminoéthoxypényl)-trans-1,2-diphénylbut-l-ène, qui est commercialisé sous l'appellation "Nolvadex". Toutefois, pour obtenir une activité antioestrogène au niveau des récepteurs oestrogéniques, notamment de la glande mammaire, l'administration per os de 10 à 30 mg par jour de ce composé est nécessaire, ce qui entraîne des effets secondaires nuisibles, notamment une stimulation paradoxale des ovaires. Ces derniers effets limitent dans une large mesure l'utilisation du tamoxifène.

On a montré en outre que le tamoxifène administré par voie orale se transforme lors de son passage hépatique en de nombreux métabolites dont le 1-(p-ß-diméthyl-amino-éthoxyphényl)-trans-1-(p-hydroxyphényl-2-diphénylbut-1-ène, ou 4-hydroxytamoxifène, qui est la forme active du produit au niveau moléculaire. En revanche, ce dérivé 4-hydroxy directement administré par voie orale serait plus vite dégradé que le tamoxifène, d'où l'inutilité de son administration par cette voie. Or, on sait également que le dérivé 4-hydroxy est de vingt à cent fois plus actif que le tamoxifène en tant qu'antioestrogène au niveau des récepteurs oestrogéniques. Toutefois, l'administration orale ou parentérale autre que percutanée aboutit à une diffusion de ce produit dans tout l'organisme, entraînant - entre autre - une stimulation paradoxale nocive des ovaires.

On a d'ailleurs décrit le dérivé 4-hydroxytamoxifène en tant qu'agent antioestrogène en vue de son administration par voie orale ou éventuellement parentérale, limitée elle-même à l'injection. Comme mentionné ci-dessus, l'administration orale paraît d'une efficacité restreinte due à la destruction du composé lui-même par l'intermédiaire du foie, tandis que l'injection, conduisant à l'introduction dudit composé dans la circulation sanguine, peut induire les actions ovariennes nocives précédemment indiquées, par effet systémique.

Le document: CHEMICAL ABSTRACTS, vol. 96, no. 9, ler mars 1982, page 29, résumé 62664k, COLUMBUS, OHIO (US), & Eur. J. Cancer clin. Oncol. 1981, 17(9), 1063-5,M. SLUYSER et al.: "Effect of monohydroxytamoxifen on mouse mammary tumors",: décrit les propriétés du monohydroxytamoxifène; sans préciser la forme cis ou trans, et dans le cadre d'une administration en pastille sous la peau et donc non percutanée.

Les travaux des demandeurs axés depuis 15 ans sur le métabolisme des stéroïdes hormonaux administrés en solution alcoolique par voie percutanée ont permis de démontrer (Journal of Clinical Investigation (USA) 1970, 49 31) que la voie d'administration percutanée des stéroïdes à demi-vie courte permettait un accès direct à l'organe cible alors que l'administration orale ou même intra-veineuse du même stéroïde privilégiait son catabolisme hépatique aux dépens de sa concentration efficace au niveau des tissus récepteurs. Le court-circuit hépatique ainsi réalisé a été démontré en premier lieu pour la testostérone (Journal of Clinical Endocrinology and Metabolism (USA) 1969, 29: 437) puis ensuite pour la progestérone (Journal of Clinical Endocrinology (USA) 1969, 29: 1590 et 1974, 38 142 et brevet FR-A-2515041). Dans le cas de la progestérone on a pu démontrer que l'administration de ce stéroïde en solution alcoolique ou en gel hydroalcoolique à 60 % permettait une rétention locozigionale de 48 heures du composé ayant franchi la barrière cutanée (10 %). Par contre par voie orale 90 % de la dose administrée est détruite dès le premier passage hépatique.

Les demandeurs ont donc été amenés dans leur travaux à essayer l'administration du dérivé 4-hydroxytamoxifène par voie percutanée afin d'éviter tout effet systémique et ils ont été surpris de constater qu'en solution alcoolique à 60 %, ce composé appliqué sur la peau recouvrant des tumeurs mammaires cancéreuses s' est révélé capable de passer la barrière cutanée et d'être capté sur les molécules réceptrices de ces tumeurs. Les demandeurs ont par contre constaté que le tamoxifène ne peut être activé en son dérivé 4-hydroxy par voie percutanée, car le sein ne dispose pas comme le foie des enzymes nécessaires à la transformation.

Le médicament antioestrogène selon l'invention, dérivant du tamoxifène, dont le produit actif est constitué par le 1-(p-ß-diméthylaminoéthoxyphényl)-trans-1 (p-hydroxyphényl)-2-phénylbut-1-ène dit 4-hydroxytamoxi-fène est présenté en un gel hydroalcoolique administrable par voie percutanée, de préférence locorégionale et pharmacologiquement acceptable.

Il est notoirement admis que le 4-hydroxytamoxifène possède - outre sa propriété de bloquer le site hormonal du récepteur des oestrogènes (action antioestrogène) - une action stimulante sur les récepteurs d'une autre hormone impliquée dans la bonne qualité trophique du sein: la progestérone. En conséquence, les demandeurs ont vérifié que l'administration concomitante par voie percutanée de 4-hydroxytamoxifène et de progestérone permet trois actions complémentaires et synergiques:

- une action antioestrogène
- la stimulation du récepteur de la progestérone
- l'occupation du récepteur de la progestérone par son hormone, ce qui amplifie son activité.

En effet, la progestérone se lie à son propre récepteur qu'elle active. Ainsi est réalisée une synergie d'action, car progestérone et oestrogène sont antagonistes au niveau de leurs

organes cibles.

Le franchissement de la barière cutanée par le 4-hydroxytamoxifène a été mis en évidence en utilisant des doses traceuses de 4-hydroxytamoxifène tritié appliqué en solution alcoolique 24 heures avant l'ablation de seins cancéreux. Les études effectuées en laboratoire ont démontré que le 4-hydroxytamoxifène était retrouvé sous sa forme originelle au niveau des structures protéiques correspondant à des récepteurs hormonaux. Il est donc capable d'avoir à ce niveau une activité antioestrogène. Une faible partie de la radioactivité est métabolisée en produits non identifiés (3 %). Parallèlement, l'on a appliqué sur la peau d'un sujet sain ce même produit sous forme radioactive et l'on a calculé la radioactivité retrouvée dans les urines dans les 15 jours suivant l'administration du produit. Le taux d'élimination urinaire montre une destruction faible et progressive du produit. Dans le sang circulant, seulement des traces du produit sont décelables-il n'y a donc pas d'accumulation. Ce n'est que secondairement qu'il atteint le foie où il est inactivé.

Au cours d'une autre expérience, des doses traceuses de progestérone tritiée furent administrées dans les mêmes conditions que le 4-hydroxytamoxifène. La progestérone a été retrouvée là encore sous sa forme originelle en partie liée aux récepteurs de la progestérone, en partie métabolisée. Dans le sang, aucune radioactivité ne circule dans les urines, différents métabolites de la progestérone sont retrouvés dans les 56 heures suivant l'expérience. On peut conclure que la progesterone est captée par des récepteurs spécifiques et qu'elle est inactivée en majeure partie in situ.

Le fait que le 4-hydroxytamoxifène et la progestérone soient solubles dans l'alcool et absorbables par la peau permet la présentation en un gel alcoolique adapté à l'administration par voie percutanée, les travaux des demandeurs faisant apparaître un coefficient d'absorption cutanée de 10 % pour la progestérone et voisin de 1 % pour le 4-hydroxytamoxifène. De manière connu en soi, le gel alcoolique comporte, outre la progestérone et le 4-hydroxytamoxifène, divers excipients nécessaires au conditionnement et permettant la pénétration percutanée, notamment du "Carbopol"®, de l'alcool éthylique et de l'eau. Les doses quotidiennes de produit à administrer sont faciles à calculer en fonction des coefficients d'absorption des médicaments et des doses que l'on veut obtenir pour le 4-hydroxytamoxifène et la progestérone au niveau de leurs molécules réceptrices.

On donne ci-après, à titre d'exemple nullement limitatif, une formulation d'un gel conforme à l'invention pour administration percutanée:

| | | |
|---|---|---|
| Progestérone | 1,5 | g |
| 4-hydroxytamoxifène | 0,15 | g |
| "Carbopol 934"® | 1 | g |
| Triéthanolamine | 1,5 | g |
| Alcool éthylique 95° | 50 | ml |
| Eau qsp | 100 | g |

(Le "Carbopol 934" ® est un polymère carboxyvinylique à groupes carboxyliques actifs, contribuant à former avec les amines des émulsions stables).

Ces produits, administrés par voie percutanée sur le sein, se concentrent électivement dans la glande mammaire et sont ensuite éliminés dans les humeurs à des taux infimes. L'effet obtenu est à l'inverse de ce que l'on observe en cas d'administration digestive où il faut obtenir une forte concentration plasmatique afin d'avoir une faible concentration locale. Dans le cas de l'administration percutanée, les taux sont maxima, près du site d'administration, minima dans la circulation sanguine et le foie. Cette technique répond donc à la demande posée: médication locorégionale antioestrogénique dans un but éventuel de traitement (maladie du sein) et ce sans effets secondaires nocifs.

La technique d'administrations locorégionale 4-hydroxytamoxifène/progestérone est donc adaptée de telle façon qu'elle réalise une optimisation des effets d'un antioetrogène sur un organe cible spécifique. Aucune autre médication ne répond à ces normes: la progestérone ne peut en effet être utilisée par voie orale car elle est totalement détruite lors de son passage par le foie.

L'association 4-hydroxytamoxifène/progestérone est capable in vitro de bloquer l'activité des oestrogènes facteurs de multiplication cellulaire et en même temps d'améliorer l'activité de la progestérone: ce sont là des actions de synergie et complémentaires que ne réalisent pas d'administration isolée de chacun des constituants de la formulation en gel mentionné ci-dessus.

La préparation du 4-hydroxytamoxifène est connue en soi et peut par exemple être réalisée selon la synthèse décrite par Robertson et Katzenellenbogen (J. Org. Chem. 1982, 47, 2387 et J. Steroïd. Biochem. 1982, 16, 1) modifiée, qui a lieu en plusieurs étapes

1) Réaction entre le 4-(ß-diméthylaminoéthoxy)-a-éthyldésoxybenzoïne et du bromure de p-(2-tétrahydropyranyloxy)-phénylmagnésium:

2) par ailleurs, formation de 1-(4-hydroxyphényl)-2-phényl-1-butanone par hydroxylation de 1,2-diphényl-1-butanone;

3) La réaction entre les produits (1) et (2) aboutit à la formation de:

1-(p-ß-diméthylaminoéthoxy-phényl)-1-[p-(2-tétrahydropyranyloxy) phényl] -2-phénylbutane-1-ol;

4) La déshydratation par méthanol-acide chlorhydrique produit le 1-(p-ß-diméthylaminoéthoxy-phényl)-1-[p-hydroxyphényl]-2-phénylbutène-1=4-OH-tamoxifène, mélange des isomères cis et trans.

5) Séparation des isomères cis et trans par chromatographie et cristallisation à activité spécifique constante.

Le médicament décrit trouve son application dans le traitement d'affections du sein, en particulier d'affections béningnes voire cancéreuses du sein.

## Revendications

1. Médicament antioestrogène, dont le produit actif est constitué par le 1(p-ß-diméthylaminoéthoxyphényl)trans-1-(p-hydroxyphényl)-2-phénylbut-1ène), caractérisé par le fait qu'il est présenté en un gel du type hydro-alcoolique administrable par voie percutanée.

2. Médicament selon la revendication 1, caractérisé par le fait que le gel comprend des excipients tels que polymère carboxyvinylique, triéthanolamine, éthanol et eau.

3. Médicament selon les revendications 1 et 2 caractérisé en ce qu'il est destiné à une application locorégionale

4. Médicament selon la revendication 1 a 3 caractérisé en ce qu'il est destiné au traitement des affections du sein en particulier d'affections bénignes voire cancéreuses du sein.

5. Médicament selon l'une des revendication 1 ou 2, caractérisé par le fait qu'il peut être associé à la progestérone conférant un effet de synergie, et présenté en un gel hydro-alcoolique administrable par voie percutanée.

6. Médicament selon la revendication 5 caractérisé en ce que dans sa formulation le rapport du produit actif à la progestérone est fonction des doses choisies pour leurs molécules réceptrices respectives.

## Patentansprüche

1. Antiöstrogenes Medikament, dessen Wirkstoff aus 1(p-ß-dimethylaminoethoxyphenyl)trans-1-(p-hydroxyphenyl)-2-phenylbut-len besteht, dadurch gekennzeichnet, daß es als ein Gel vom hydroalkoholischen Typ dargereicht wird, das auf perkutanem Wege verabreicht werden kann.

2. Medikament gemäß Anspruch 1, dadurch gekennzeichnet, daß das Gel Exzipiente, wie Carboxyvinyl-Polymer, Triethanolamin, Ethanol und Wasser aufweist.

3. Medikament gemäß Anspruch 1 und 2, dadurch gekennzeichnet, daß es für eine locoregionale Anwendung bestimmt ist.

4. Medikament gemäß Anspruch 1 bis 3, dadurch gekennzeichnet, daß es für die Behandlung von Erkrankungen der Brust bestimmt ist, insbesondere für gutartige und sogar kanzeröse Erkrankungen der Brust.

5. Medikament gemäß einem der Ansprüche 1 oder 2, dadurch gekennzeichnet, daß es zusätzlich zu Progesteron verabreicht werden kann, wobei es einen Synergie-Effekt hervorruft, und daß es als hydroalkoholisches Gel dargereicht wird, das auf perkutanem Wege verabreicht wird.

6. Medikament gemäß Anspruch 5, dadurch gekennzeichnet, daß das Verhältnis von Wirkstoff zu Progesteron in der Formulierung von den Dosen abhängig ist, die für ihre jeweiligen Aufnahmemoleküle gewählt werden.

## Claims

1. Anti-estrogen drug, in which the active product consists of 1-[p-(ß-dimethylaminoethoxy)phenyl]-trans-1-(p-hydroxyphenyl)-2-phenylbut-1-ene, characterized in that it is presented as a gel of the aqueous alcoholic type which can be administered percutaneously.

2. Drug as claimed in claim 1, characterized in that the gel contains excipients such as carboxyvinyl polymer, triethanolamine, ethanol and water.

3. Drug as claimed in claims 1 and 2 characterized in that it is intended for locoregional application.

4. Drug as claimed in any one of claims 1 to 3 characterized in that it is intended for the treatment of conditions of the breast, especially benign and even cancerous conditions of the breast.

5. Drug as claimed in any one of claims 1 or 2 characterized in that it is, in addition, combined with progesterone conferring a synergistic effect, and presented as an aqueous alcoholic gel which can be administered percutaneously.

6. Drug as claimed in claim 5 characterized in that the ratio in the formulation of the active product to progesterone depends on the doses chosen for their respective receptor molecules.